# EUROPEAN PATENT APPLICATION

(11) **EP 4 693 203 A1**
(43) Date of publication of application: **11.02.2026**
(21) Application number: 24192904.1
(22) Date of filing: 05.08.2024
(51) Int. Cl.: G06T 15/20

(54) **METHOD AND SYSTEM FOR ESTIMATING AN OPTIMAL DENTAL SITE VIEW**

(71) Applicant: 3Shape A/S, 1060 Copenhagen K (DK)
(72) Inventor: CEBOV, Aleksandar, 1060 Copenhagen K (DK); HUSEINI, Admir, 1060 Copenhagen K (DK); AVRAMOSKI, Kiril, 1060 Copenhagen K (DK); KADIEV, Kostadin, 1060 Copenhagen K (DK)
(74) Representative: Guardian IP Consulting I/S

(57) **Abstract**

The disclosure relates to computer-implemented method and system for generating an optimal view direction of an affected area in a 3D model of intra-oral scan data allowing a dental health care professional to inspect and convey dental assessments in an efficient, fast and optimized manner. In more detail the method and system described herein is configured to estimate the optimal view from which an affected area of a dental site may be displayed on a graphical user interface while allowing automated updates of the view direction to bring the affected site into focus of a graphical user interface.

## Description

### FIELD

The disclosure relates to computer-implemented method and system for generating an optimal view direction of an affected area in a 3D model of intra-oral scan data allowing a dental health care professional to inspect and convey dental assessments in an efficient, fast and optimized manner.

### BACKGROUND

Digital dentistry is becoming increasingly popular and offers several advantages over non-digital techniques where especially utilizing digital dentistry to assert diagnostic measures of an oral cavity and evaluate treatments possibilities is becoming increasingly relevant. Within digital dentistry intra-oral scan data may be obtained, e.g. using an intra-oral scanner, from which data a 3D model may be generated upon processing the intra-oral scan data. The generated 3D model reflects the dental health situation of a patient and may be processed to generate a diagnostic assessment of the 3D model, allowing a dentist to assess the health status of the oral cavity of a patient. The oral cavity in general refers to the mouth, and may include one or more of the lips, the lining inside the cheeks and lips, the front two thirds of the tongue, the upper and lower gums, the floor of the mouth under the tongue, the bony roof of the mouth, and the small area behind the wisdom teeth as well as the teeth in general.

When performing digital diagnostic assessments or at least providing solutions aiding the dentist or other dental health care professionals in assessing the processed 3D model, it is important that the findings associated with e.g. different dental conditions are processed and presented to the dentist in a precise and accurate manner allowing efficient communication with the patient. Today's methods for aiding in diagnostic assessment of oral cavities often rely on manually navigating for example a 3D dental model in a user interface, where the manual navigation relies on a touch, click or other screen input on the user interface, which controls the navigation of the 3D model in the user interface. Accuracy and orientation of the areas presented to e.g. a patient on a display of a computer relies strongly on the manual navigation by the dentist and may result in slow navigation of the 3D model between areas of interest, such as dental condition lesions as well as inaccurate views of the areas of interest in view of potentially identified dental conditions.

Accordingly, a need exists for solutions which allow a dentist or other dental health care professional to inspect dental conditions in an oral cavity in a fast, accurate and efficient way optimizing the time spent per patient and allowing efficient communication with the patient.

### SUMMARY

The present disclosure addresses the above-mentioned challenges by providing a computer-implemented method for generating an optimal view direction of an affected area in a 3D model of intra-oral scan data. The method comprises rendering a 3D model in a graphical user interface of a computer display, wherein the 3D model comprises one or more affected areas. Receiving by a processor a user input to the graphical user interface, wherein the user input encodes a position representation in the rendered 3D model, and retrieving the position representation and determining if the position representation is associated with an affected area. Furthermore, if the position representation is associated with an affected area, the method comprises determining an optimal view direction of the affected area using the position representation. The optimal view direction may be found according to the method by determining a surface normal of the position representation, estimating the reverse normal of the surface normal, and setting the optimal view direction as the reverse normal and updating the 3D model view in the graphical user interface to correspond to the set optimal view direction. The determination of an optimal view direction as described herein ensures that the 3D model is updated to a view position from where a selected affected area as provided from the user input may be seen most directly. This allows the dentist to efficiently assess the area affected and determine if potential treatments are relevant for that area as the method provides for an automatic determination of the best view direction of an affected area and to visualize the selected affected area from all angles.

Accordingly, the features supporting the method of estimating the optimal view direction assist the user (such as any dental professional) in performing a dental assessment task in an effective manner as the method supports the fast speeded analysis of a dental situation by automatically defining for the user the optimal view of the affected area. The automatic updating of the 3D model based on the estimated optimal view direction furthermore assists the user in an improved and more efficient human-machine interaction process, that allows fast assessment of the dental situation. This may facilitate a more efficient analysis leading to a more efficient treatment of affected areas of a dental situation of a patient.

The position representation may, according to the method described herein, encode for a selection of at least one facet of the 3D model. Accordingly, the 3D model may comprise a plurality of facets representing teeth and/or gingiva in the 3D model, wherein the plurality of facets combined provides for a 3D mesh model.

Alternatively, the position representation may according to the method encode for a selection of at least one point in the 3D model. Accordingly, the 3D model may comprise a plurality of points representing teeth and/or gingiva, wherein the plurality of points provides a 3D point cloud representation of the 3D model. That the position representation may encode for a selection may be understood that the selection may comprise a selection of one or more points, areas or other areas of interest in the oral cavity that are processed in a processor to perform the method described herein.

To ensure that the position representation forms part of an affected area, the method comprises evaluating if the selected at least one facet forms part of the affected area of a tooth and/or a gingiva area of the 3D model.

If the 3D model is represented as a point cloud, the method comprises evaluating if the selected at least one point, or preferably set of points, forms part of the affected area of a tooth and/or gingiva area of the 3D model. In any of these examples, the checking of whether the position representation in the form of a point, plurality of points or at least one facet forms part of the affected area, ensures that the optimal view direction is only determined by a processor executing the method, if the position representation is associated with an affected area. This ensures that unnecessary processing of the 3D model is avoided as this processing step ensures that the 3D model view is updated only when an affected area has been chosen by the user. In this way, the method ensures that the dentist avoids presenting to e.g. a patient or asserting a non-affected area which is not of relevance for any further treatment processes.

To avoid presenting a non-affected area, which was potentially unintentionally selected from a user by the user input, the method comprises the previous mentioned evaluation and further to evaluating if the facet or previously mentioned point(s) does not form part of an affected area, and if so the method is configured to de-select the facet or point selected by the user. This may further cause the processor to execute the step of re-orienting the view direction of the 3D model to a center of location of a global coordinate system of the 3D model.

The dental situation as described herein may comprise an upper and lower jaw representation of a patient, where the upper and/or lower jaw may be affected by one or more health changes that may cause a reason for treating one or more affected areas in the dental situation. The one or more health changes may comprise for example one or more dental conditions, such as caries, plaque, wear, crack, recession, gingivitis, pocket depths and/or inflamed gingiva amongst other dental health situations. The dental condition may be the root causes for treatments applied to teeth and/or gingiva of the dental situation, where treatments applied prior to a dental scan may include one or more of fillings, veneers, inlay-onlays, bridges, crowns, brackets etc. Each of these treatments may be present in the 3D model as generated and rendered as described herein. Accordingly, the affected sites as described herein, may comprise any of the one or more dental conditions and/or one or more of the treatments applied, allowing a dentist to assess both detected dental conditions as well as treatments applied to the dental situation using the method described herein.

As the 3D model represents a full jaw comprising the upper and lower jaw, the affected area may be detected and assessed according to the method described herein on a buccal, lingual and/or occlusal side of a tooth and/or gingiva area of the 3D model. The estimation of the optimal view direction accordingly allows to assert all relevant sites of the dental situation.

The surface normal described herein may be calculated from at least one facet or at least one or more points representing a surface behavior of an affected area. The reverse normal may subsequently be estimated from the calculated surface normal.

In one embodiment, the facet normal is determined as a 3D vector arranged perpendicular to a facet surface, where in a facet is represented by a triangular geometrical structure comprising vertices connecting edges of the vertices. The geometrical structure may also be configured as a polygon. In an embodiment where the 3D model is configured as a point cloud, the surface normal may also be calculated from obtaining an underlying surface from the 3D model point cloud dataset using for example surface meshing techniques and subsequently computing the surface normals from the underlying surface. Alternatively, the surface normal may be determined by using approximations by for example using a principal component analysis to approximate the surface normal directly from the point cloud dataset directly.

In an embodiment, the method may take into account a group of facets or points associated with an affected area to calculate the surface normal used to estimate the reverse normal allowing the setting of the optimal view direction. In accordance herewith, the affected area comprises one or more facets (or points), wherein setting the optimal view direction comprises determining surface normals for the one or more facets or points forming part of the affected area. This may be done by calculating an average of the surface normals and estimating the reverse normal of the averaged surface normals. The method further comprises setting the optimal view direction as the reverse normal and updating the 3D model view in the graphical user interface to correspond to the set optimal view direction. This is ensuring that more than one single facet or point of an affected area is taken into account when determining the optimal view direction and allows for a more accurate view direction and thereby representation of the entire affected area when viewed with the 3D model.

In an embodiment, the reverse normal vector may be determined by normalizing the averaged surface normals from the affected area facets and multiplying with -1.

To ensure that a sufficient amount of the affected area is comprised in the estimation of the optimal view direction of the affected area, setting the optimal view direction may further comprise determining facets or points surrounding the affected area to determining the average of the surface normals within the affected area and the surface normals of facets surrounding the affected area. The surrounding facets or points may be determined by applying a flood filling approach to calculate a distance between facet mid points of facets or points surrounding the affected area to facets or points within the affected area and applying a set threshold to determining if a surrounding facet or point should be included in the averaging or not.

In an embodiment, the affected area may comprise a boundary encircling the affected area, wherein the boundary comprises control points. The encircling of the affected area may be represented on the 3D model to allow a clearer identification of an affected area as determined by detection algorithms, wherein the detection algorithms for example detects automatically one or more dental conditions, one or more fillings, crowns, bridges, brackets etc. in the dental situation. The encircling of the detected area by a boundary ensures easy visual inspection of the 3D model by a dentist and assists the dentist in quickly getting an overview of the affected areas thereby optimizing the treatment time spent per patient.

To ensure that an affected area is not occluded by other parts of the dental site and thereby ensuring that the dentist can see the entire affected area in full, the method comprises determining an area affected occlusal view. The area affected occlusal view may be configured as a non-affected area of for example tooth and/or gingiva or other parts of the dental site causing occlusion of at least a part of the affected area. According an embodiment of the method the area-affected occlusal view may be determined by determining one or more boundary points of the affected area and estimating a ray from at least one boundary point and determining if the set optimal view direction is occluded by the non-affected area of tooth and/or gingiva. The ray estimated from a boundary point may be directed towards a direction where an occlusion appears when the estimated ray is seen from the optimal view direction. When the estimated ray is not occluded by a part of the dental situation when seen from the set optimal view the direction, the method comprises returning the set optimal view direction if no occlusion is determined.

If on the other hand the estimated ray from a boundary point is found to be occluded by a part of the dental situation when the ray is seen from the set optimal view direction, the method may in an embodiment comprise adjusting the set optimal view direction to ensure that the boundary point can be seen from the adjusted set optimal view direction.

Alternatively (and/or in combination with the previous described embodiment), if the estimated ray from a boundary point is found to be occluded by a part of the dental situation, such as for example a non-affected area of tooth and/or gingiva, the method may comprise applying a transparency setting to the non-affected area of tooth and/or gingiva (or any other part of the dental situation occluding the view of the affected area), wherein the transparency setting adjusts the opacity of the 3D model at the non-affected area of tooth and/or gingiva or any other part of the dental situation occluding the view of the affected area. In this way it is ensured that if an occluded area is found and for example the optimal view direction cannot be set to accurately show the affected area in full, the method applies for a solution allowing a dentist to inspect the full area despite the occluded view by using the mentioned automatically set transparency setting.

As the dentist when assessing the affected area is likely to interact with the 3D model by rotating, panning, zooming etc. the 3D model, the method described herein ensures that such interaction with the 3D model is sufficiently supported to ensure that the affected site always can been seen in the most optimal manner. That is the method may further comprise setting a center of rotation of the affected area by determining a bounding box of at least one tooth enclosing all facets of the affected area and calculating the center of the tooth and setting the center of the tooth as the center of the affected area. This ensures that a dentist upon inspection and potentially conveying of dental information to a patient regarding an affected area is assisted by the method so that the affected area always stays in the optimal view direction even when rotating, zooming or panning the model to show for example different sides of the affected area.

In an alternative the setting of the center of rotation may comprise utilizing a center of mass of the affected area. In an alternative, the setting of the center of rotation may also comprise setting the center of rotation to a surface point of the affected area selected by a user.

To further assist the dentist in assessing affected areas and/or conveying the information effectively to a patient, the method may comprise generating an area affected control and representing the area affected control in the graphical user interface, wherein the area affected control is generated upon receiving a user input to a facet or point of the affected area, and wherein the area affected control is configured to display tooth number, diagnostic indication and/or a severity of a diagnostic indication associated with the facet of the affected area. Furthermore, the area affected control may be configured with crown information, bracket information, filling information, bridge information or any other relevant treatment information that concerns the affected area chosen. With this, the method aids the dentist by providing a summary view of for example a single tooth or gingiva affected area allowing a dentist to assess to specific chosen area in a fast an efficient manner and at the same time conveying the information to a patient. As previously mentioned, the user selected area may also be construed as a point and/or a group of points representing the selected affected area.

The user input may be configured as an action of a user according to a voice input, eye tracking input and/or for example gyro input when for example the intra-oral scanning device is configured to be used as a pointing device.

To ensure that a dentist may change a parameter associated with an affected area, such as type of dental condition, severity of dental condition, type of previous treatment that has been detected etc., the method comprises ensuring that the area affected control is configured with interactive elements which may be used by the dentist to for example change a parameter of the affected area. Accordingly, the method comprises receiving at least one user input to an interactive element, wherein the user input encodes a change parameter to the diagnostic indication and/or severity. The method is configured to retrieve the change parameter by the processor and updating the diagnostic indication and/or severity in accordance with the change parameter. Furthermore, the method ensures that the change is subsequently updated on the 3D model, as the method comprises displaying an updated representation of the affected area comprising the diagnostic indication and/or severity on the 3D model in the graphical user interface. This ensures that the dentist can easily inspect the affected area using the previous described method steps by the automatic estimation of the optimal view direction and subsequently apply any relevant change to the affected area which the dentist finds relevant in view of e.g. a change to a severity of a detected dental condition, or for example a change to a filling area, a crown area etc. In this way the dentist is aided in creating a fast working environment that allows efficient treatment processes of patients. By providing this change mechanism, the dentist is void from for example disregarding the 3D model findings if the found affected areas are not correctly identified by the automated detection algorithms, as the dentist may easily change the findings of the affected areas to a correct parameter in view of the actual clinical assessment performed by the dentist when evaluation the dental situation using potentially both the 3D model and a clinically visual inspection of the intra oral dental situation in the mouth of a patient.

As previously explained, the user input to the interactive element of the area affected control, may be a change in diagnostic indication of the affected area and/or a change in severity, and the change parameter may encode for a color change to the affected area in the 3D model. The color may indicate for example a severity of a dental condition, wherein a first severity may be encoded with a 1^{st} color, a second severity may be encoded with a 2^{nd} color and one or more severities advancing the 1^{st} and 2^{nd} severity may be encoded with one or more different colors. The change parameter may also be encoded as a pattern, where each different pattern encodes for a different severity and/or affected area finding. Distinguishing dental condition severities by color and/or other parameters of the affected area by color or pattern, this area affected control allows the dentist to change the parameter into a different type of severity or parameter of the affected area in a fast and efficient manner speeding up the clinical assessment of the dental situation. One change parameter may encode for a deletion of the affected area, in case the dentist evaluation of the automated detection of the affected areas results in an evaluation of the affected area being wrongly detected as an affected area. In such case, the change parameter may for example mark the affected area or at least the boundary of the affected area in for example a white color indicating to the dentist and patient, that the changed area of the 3D model is not considered clinically relevant affected area.

To ensure that the area affected control does not obstruct the view of the 3D model and the area affected sites, the method comprises generating the area affected control in close proximity to and/or connected to the selected affected area.

Further, to ensure that the area affected control does not obstruct the affected area when the dentist engage interactively with the 3D model by for example rotating, zooming of panning the 3D model or the affected area the method comprises arranging the area affected control in a non-occluding manner to the selected affected area. That is the area affected control may be rendered in the scene of the 3D model in relation to the affected area in a non-occluding manner of the selected affected area.

In more detail, the method therefore comprises generating the affected area control by utilizing control points of the affected area boundary and positioning the affected area control at the right most control point of the affected area boundary, wherein upon rotation, zoom or pan of the 3D model, the affected area control position is updated to a new right most control point of the area affected boundary. This ensures that the affected area control is always rendered at an outer-most position of the affected area and thereby ensures that the affected area may not be occluded by the affected area control. It should be understood, that the mentioned right most position might as well be configured as a left, top or bottom most position.

The method described herein may form part of a system as a whole. In an example the system may configured for generating 3D models and inspecting diagnostic data, such as dental conditions and or other affected areas, such as previous performed treatment of a dental situation. The system may comprise an intra-oral scanner configured to obtain scan data representing the intra-oral cavity of a patient. The system may further comprise a computing device operatively communicating with the intra-oral scanner wherein the computing device may be configured to generate a 3D model of the intra-oral scan data. As previously mentioned, the 3D model may represent a plurality of data points representing a jaw, such as the upper and lower jaw of a patient. The plurality of data points may be in the form of meshes represented by a plurality of facets or may be in the form of a plurality of points representing a point cloud.

The computing device may furthermore be configured to identify one or more affected areas in the intra-oral scan data. The one or more affected areas may be a dental condition, such as a caries lesion, a plaque site, a recession site, a tooth wear condition, gingival inflammation site and/or other dental conditions associated with an intra-oral cavity. Alternatively, and/or additionally, the affected area may also comprise one or more of a filling, a crown, a bridge or any other treatment previously performed to the dental situation prior the obtaining of the scan data.

The computing device may furthermore be configured to render a 3D model from the generated 3D model of intra-oral scan data in a graphical user interface of a computer display, wherein the 3D model comprises one or more affected area. To allow the dentist to inspect and convey the affected area findings to a patient the computing device may furthermore be configured to receiving by a processor a user input to the graphical user interface, wherein the user input encodes a position representation in the rendered 3D model. As previously explained, this allows a user to efficiently select an affected area for inspection, and to ensure that the affected area is actually chosen, the computing device is configured to retrieving the position representation and determining if the position representation is associated with an affected area. If the position representation is associated with an affected area, then the computing device is configured to setting an optimal view direction from where the affected area is displayed in the graphical user interface using the position representation. This ensures that the 3D model is rendered in the graphical user interface from a direction at which the affected area may best be seen. The setting of the optimal view direction may comprise determining a surface normal of the position representation and estimating the reverse normal of the surface normal. Subsequently, the computing device is configured to setting the optimal view direction as the reverse normal and updating the 3D model view in the graphical user interface to correspond to the set view direction.

As the system described herein is configured to perform the method described herein and as reflected above, it should be understood that the method steps described throughout also applies for the application of the system performing the method. Additionally, the different mentioned alternative embodiments in relation to the described method equally applied for the system described and are considered to be combinable with the mentioned system features.

In an embodiment, the scan data described herein may comprise at least one of fluorescence information, a color information, a depth information, a texture information, and/or an infrared information. The scan data may also comprise data associated with CBCT, X-ray or other suitable intra-oral cavity scan data.

Furthermore, the computing device may in an embodiment comprise a data processing system comprising means for carrying out the method described herein.

In an embodiment the data processing system may be configured to perform the method at a cloud processing server and transmit the method outputs to a local server of a computing device. Alternatively, the data processing system may be configured to perform the method on a local server of a computing device.

Furthermore, the disclosure provides for a computer program product embodied in a non-transitory computer readable medium comprising computer readable program code configured to be executed by a hardware processor to cause the hardware data processor to perform the methods disclosed herein when the computer readable program code is executed by the hardware data processor.

### BRIEF DESCRIPTION OF THE FIGURES

Examples described herein may be best understood from the following detailed description taken in conjunction with the accompanying figures. The figures are schematic and simplified for clarity, and they just show details to improve the understanding of the claims, while other details are left out. The individual features of each example described may each be combined with any or all features of the other examples unless stated otherwise. These and other examples, features and/or technical effect will be apparent from and elucidated with reference to the illustrations described hereinafter in which:
Figure 1 illustrates a dental scanning system according to the disclosure;
Figure 2 illustrates a dental scanning system of an intra-oral scanner and a computing device having a display unit with a graphical user interface;
Figure 3 illustrates a 3D model with an exemplified data point representation;
Figure 4 illustrates a 3D model with detected affected areas and a position representation of a selected affected area from a user input;
Figure 5 illustrates a surface normal of a position representation of a selected affected area;
Figure 6 illustrates a reverse normal of the position representation according to Figure 5;
Figure 7 illustrates a group of surface normals associated with an affected area;
Figure 8 illustrates and averaged surface normal associated with an affected area;
Figure 9 illustrates a 3D model with an affected area comprising a surface normal, with the 3D model in an out of focus view of the affected area;
Figure 10 illustrates the 3D model of Figure 9, where the 3D model view is updated bringing the affected area into a focused view;
Figure 11 illustrates a plurality of estimated rays from control points of boundary of affected area;
Figure 12 illustrates a 3D model with an affected area, where parts of the affected area is occluded by teeth;
Figure 13 illustrates the 3D model of Figure 12, where an opacity setting has been applied to the area of the tooth occluding the affected area;
Figure 14 illustrates a 3D model with a first position of the affected area control;
Figure 15 illustrates the 3D model of Figure 14, where the affected area have been updated to a new position as a result of a rotation of the 3D model;
Figure 16 illustrates selection of an affected area on a 3D model;
Figure 17 illustrates updating the 3D model of Figure 16 into an optimal view direction of the selected affected area;
Figure 18 illustrates the affected area control of a 3D model;
Figure 19 illustrates a change to the affected area based on an interaction with the affected area control of Figure 18; and
Figure 20 illustrates a flowchart of the method of setting the optimal view direction described herein.

### DETAILED DESCRIPTION

The detailed description set forth below in connection with the appended drawings is intended as a description of various examples according to the disclosure. The detailed description includes specific details for the purpose of providing a thorough understanding of various concepts and examples covered throughout the disclosure. However, it will be apparent to those skilled in the art that these concepts and examples may be practiced without the specific details mentioned or in combination with one or more examples described herein. Several examples of the devices, systems, media or mediums, programs and methods are described by various modules, components, steps, processes, algorithms, etc. Depending upon particular application, design constraints or other reasons, these elements may be implemented using electronic hardware, computer program, or any combination thereof. In the following several examples of the methods and system described herein will be disclosed in more detail.

In the following detailed description, the affected area of the dental model may be described as a lesion affected area and/or a other types of affected areas such as previous treatment affected areas. Furthermore, the term "affected area" may be interchangeable used with the term "affected site" and should be construed to mean the same. It should be understood that the method described herein could be applied to asses dental affected areas, such as attachments, fillings, crowns, roots, veneers, brackets or any other relevant affected site that could be of relevant to asses in further detail on a 3D model of a dental situation as well as the assessment of detected dental conditions.

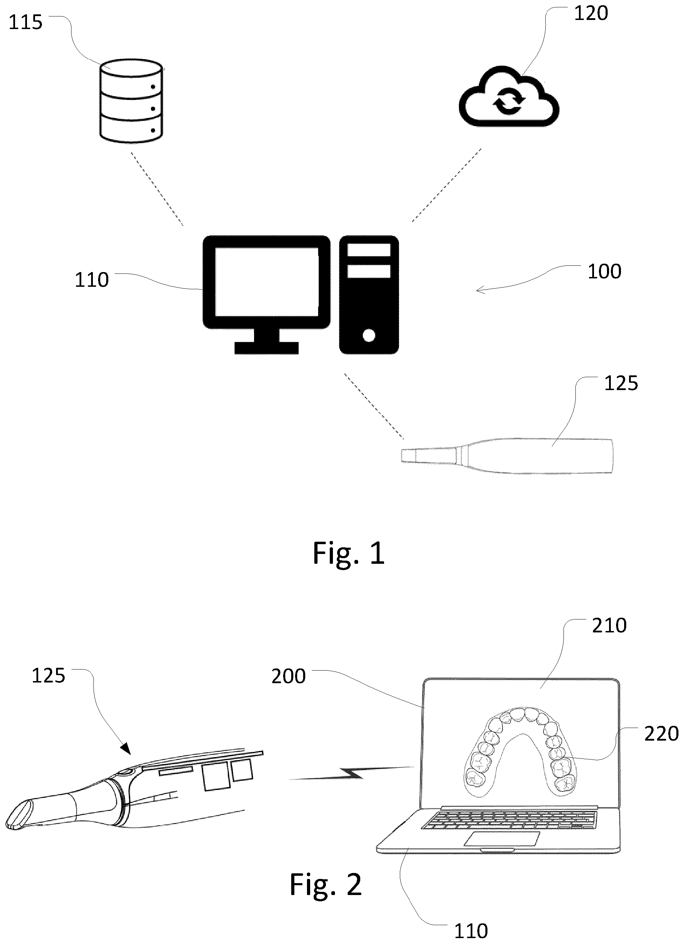

Referring to Figure 1 a dental scanning system 100 capable of performing the method according to embodiments described herein is illustrated. In more detail, Figure 1 illustrates a dental scanning system 100 comprising a computer 110 capable of carrying out any method described herein. The computer 110 may comprise a wired or a wireless interface to a server 115, a cloud server 120 and an intraoral scanner 125. The intraoral scanner 125 is configured to recording scan data comprising geometrical information, natural color information, infrared information and/or fluorescence information associated with the patient's dentition. The intraoral scanner 125 is configured with various modules such as a fluorescence module and/or an infrared module and thereby capable of capturing information relevant for diagnosing dental conditions such as caries, dental recession, tooth cracks, gingivitis, gingival inflammation, periodontal diseases, oral cancerous regions and/or plaque.

The dental scanning system 100 may comprise a data processing device configured to carry out the method according to one or more examples described herein. The data processing device may be a part of the computer 110, the server 115 or the cloud server 120.

As seen in Figure 2, computer 110 of the dental scanning system 100 comprises a graphical user interface 210 of a display unit 200. The display unit 200 can be a computer screen, a touchpad screen or e.g. a smart phone screen configured as a part of the computer 110. As seen in Figure 2, the scan data obtained by the intra oral scanner 125 may be processed by either the server 115, the cloud server 120 or a processor of the computer 110 to generate a 3D digital model 220, which can be rendered in the graphical user interface 210 of the display unit 200.

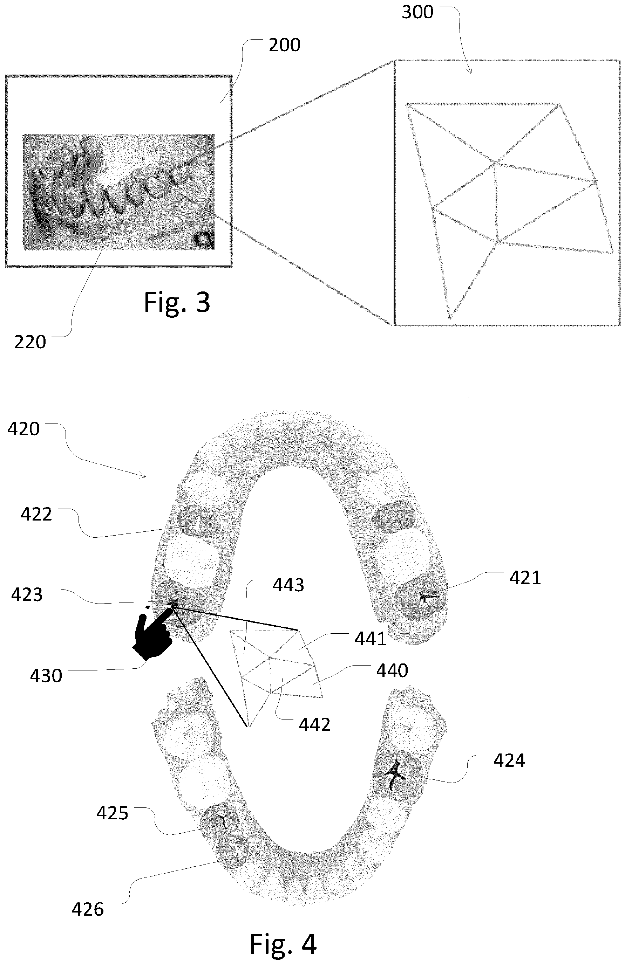

Illustrated in Figure 3 is an example of a three-dimensional (3D model) 220 on a display unit 200. As seen in Figure 3, the 3D model 220 may represent teeth and gingiva of a patient as acquired by an intra-oral scanner 125 during an intra-oral scanning session at e.g. a dental clinic. The intra-oral scanner obtains as previously mentioned scan data, which may be processed to generate the 3D model 220. In one example, the 3D model 220 generated from the scan data comprises a plurality of facets 300 which represents teeth and gingiva of an intra-oral cavity. The facets 300 may be considered as a plurality of triangles connected to each other, where each facet may be represented by at least three points. In another example the 3D model may be represented as a point cloud instead of facets.

When a patient visits a dental clinic, using digital intra-oral scan techniques, a digital 3D dental model may be acquired, where the 3D dental model may be used to asses an oral health status and to provide a clinical assessment of oral health. The clinical assessment comprises amongst other clinical assessment to determining potential dental conditions present in the patient's oral cavity, where the dental conditions may include caries, plaque, cracks, recession, tooth wear, malocclusion, recession, gingivitis, cancerous regions and/or similar conditions associated with dental health. As previously explained, the output from a scan secession using an intra-oral scanner may as seen in Figure 2 be the obtaining of a 3D model 220 of teeth and gingiva of the patient, which are rendered into the display 200 of a graphical user interface 210. To facilitate evaluation of the oral situation as well as providing communicative explanations to the patient, the 3D model 210 is presented to the user (i.e. both dentist and patient) on the display unit 200, where the method described in the following provides a method of supporting an optimized, automatic and efficient way of assessing and conveying information on oral health to a patient.

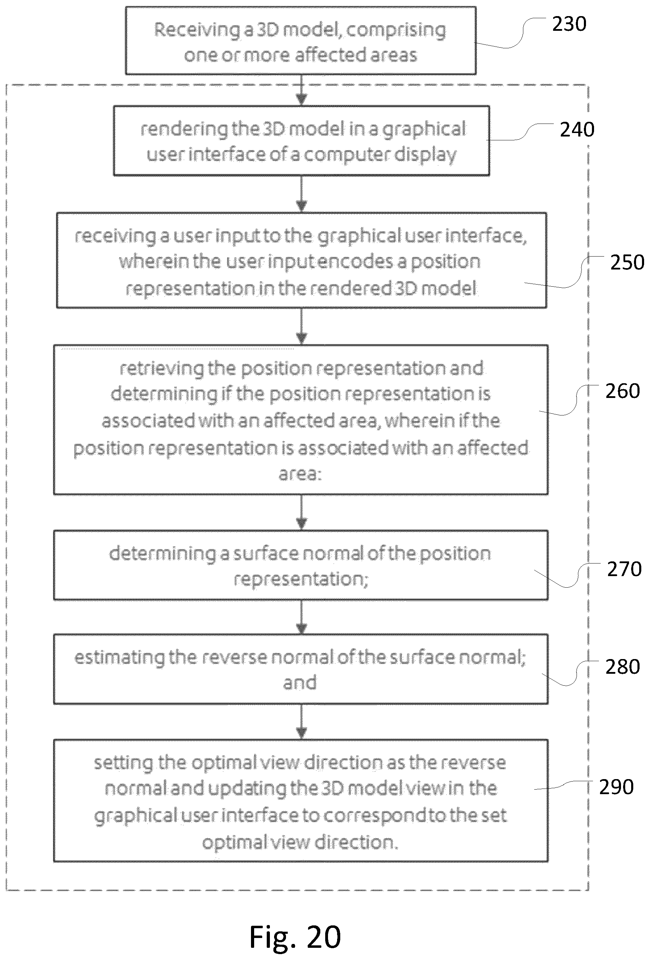

Accordingly, and with reference to Figure 4 and Figure 20, the method described herein utilizes a processor on a cloud, server or on the computing device to rendering a 3D model of a patient on the graphical user interface, wherein the 3D model comprises intra-oral scan data information of detected one or more dental conditions. The detection of the dental conditions may be a result of automated analysis of the scan data using e.g. trained neural networks and/or algorithmic procedures. The conveying of information to both dentist and patient is with the method described herein assisted by automated processes allowing a dentist to easily navigate the 3D model areas on which the dental conditions have been detected. In more detail, the automated processes described herein ensure that accurate information sharing by providing an optimized view of the conditions detected is provided for. Accordingly, to aid the dentist in efficiently evaluating, understanding and communicating a dental condition assessment to a patient by especially providing optimal views on the affected areas of the 3D model when navigating the model, the method described herein suggest providing a computer-implemented method for generating an optimal lesion view in a 3D model of intra-oral scan data, wherein the method comprises:
- rendering 240 a 3D model 420 in a graphical user interface of a computer display, wherein the 3D model comprises one or more lesion affected areas 421, 422, 423, 424, 425, 426 as illustrated in Figure 4.
- receiving 250 by a processor a user input 430 to the graphical user interface, wherein the user input 430 encodes a position representation 440; 441; 442; 443 in the 3D model 420;
- retrieving 260 the position representation 440; 441; 442; 443 and determining if the position representation is associated with a lesion affected area, exemplified as area 423 in Figure 4 as the chosen lesion affected area 423 by the user input 430. Other lesion affected areas are in Figure 4 exemplified by lesions affected areas 421, 422, 424, 425, 425. The method then evaluates if the position representation 440; 441; 442; 443 is associated with a lesion affected area 423, whereby the method comprises:
- determining 270, 280, 290 an optimal lesion view direction using the position representation by:
   - determining 270 a surface normal of the position representation;
   - estimating 280 the reverse normal of the surface normal; and
   - setting 290 the optimal lesion view direction as the reverse normal and updating the 3D model view in the graphical user interface to correspond to the set lesion view direction.

The method steps are illustrated in Figure 20 in a logical flow chart, and comprises receiving 230 a 3D model comprising one or more affected areas; rendering 240 the 3D model in a graphical user interface of a computer display; receiving 250 a user input to the graphical user interface, wherein the user input encodes a position representation in the rendered 3D model; retrieving 260 the position representation and determining if the position representation is associated with an affected area, wherein if the position representation is associated with an affected area determining 270 a surface normal of the position representation; estimating 280 the reverse normal of the surface normal; and setting 290 the optimal view direction as the reverse normal and updating the 3D model view in the graphical user interface to correspond to the set optimal view direction.

It should be noted that the computer display may also be configured as a smartphone display, a hologramic display or for example augmented and virtual reality glasses

By determining an optimal lesion view using a surface normal of the position representation and estimating a reverse normal that can be used to setting the optimal lesion view it is ensured that the 3D model is updated efficiently and accurately to the direction of view from which a lesion affected area may be seen most directly and from all angles. In this way the user, such as a dentist, can easily chose a lesion affected area by a simple click or touch on the display wherein the graphical user interface has the 3D model rendered, whereby the method as described herein automatically determines the surface normal and reverse surface normal and subsequently updating the 3D model view in the graphical user interface in accordance herewith.

With reference to Figure 4, an example of a 3D model 420 as output from one or more dental condition detection algorithms is illustrated. The 3D model 420 of Figure 4 may be considered to be displayed in the display unit 200 as for example explained in relation to Figure 2 or Figure 3. The 3D model 420 rendering in a display, may be interactively operated and/or assessed by for example clicking, panning, zooming or other perform other relevant interaction with the model. Any interaction with the model is considered to encode for an action of a processor to for example rotate, zoom, pan or selecting certain areas in the model, where especially selection of e.g. an affected area may according to the method described herein encode for a position representation. Furthermore, the 3D model 420 is rendered in the graphical user interface and comprises one or more affected areas 421, 422, 423, 424, 425, 426. Each of the affected areas illustrated in Figure 4 represents for example a dental condition detected by one or more automated processes, and in the specific non-limiting example of Figure 4, the affected area could for example be caries lesions of different severity as indicated by the slight difference in colors of the lesions in the given example.

To facilitate a method allowing a dentist to communicate efficient and accurate information to a patient, the method comprises receiving a user input 430 to the graphical user interface 210, represented in Figure 2 where the 3D model 220, 420 has been rendered. The user input 430 may be that of a click of a mouse, a touch on the screen or any other suitable interaction with the user interface 210. The user input 430 encodes for a position representation such as a facet or point selection in the 3D model 220, 420. With reference to the example shown in Figure 4, this means that at least one of the facets, exemplified by facets 440, 441, 442, 443 representing the affected area 423 may be configured as the position representation that the user input 430 encodes for. When the processor receives the position representation being e.g. a facet selection 440, 441, 442, 443 (or alternatively a point selection in case of the 3D model being represented as a point cloud), the method comprises retrieving the position representation 440, 441, 442, 443 and determining if the position representation is associated with the affected area 423.

To ensure that the 3D model view direction is updated in accordance with the position representation selection, the method comprises determining a surface normal of the position representation and estimating the reverse normal of the surface normal. The method then is configured to setting the optimal view direction as the reverse normal and subsequently updating the 3D model view in the graphical user interface to correspond to the set optimal view direction. This ensures that the orientation of the 3D model in relation to a virtual camera of the graphical user interface is updated to create the most optimal view on the affected area.

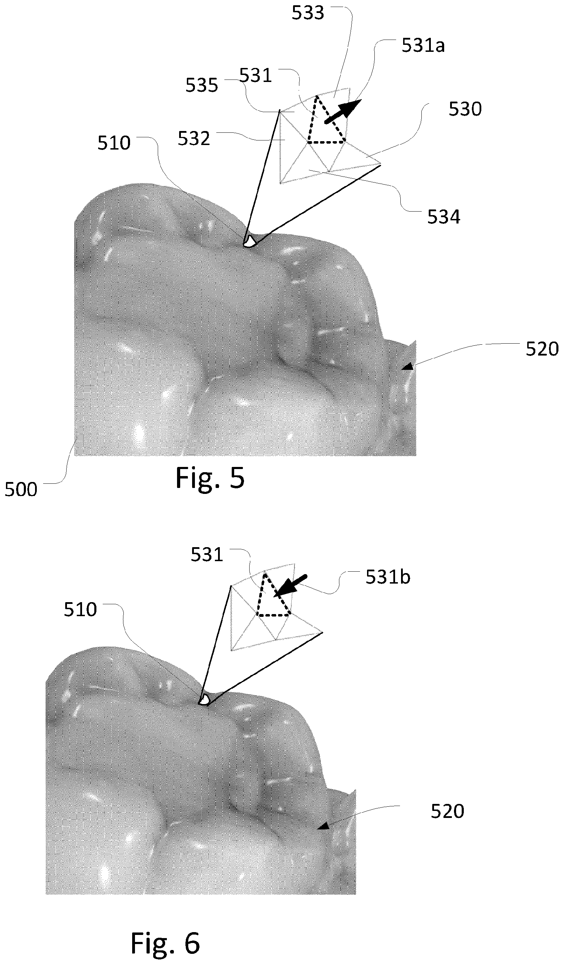

With reference to Figure 5, the method steps of determining an optimal view direction using the position representation is described in an example. Consider that tooth 500 forming part of a 3D model 520 upon which one or more dental conditions have been detected, comprises an affected area 510, which is represented by a plurality of facets 530, 531, 532, 533, 534, 535 that are enclosed in the affected area 510 is of interest for a dentist to inspect and convey to a patient. In this case, the dentist may from the graphical user interface select the affected area 510, whereby the method comprises determining if the selected affected 510 in the form of a position representation is associated with the affected area 510. If in this case, the selected facet 531 is associated with the affected area 510, then the processor is configured to determine the surface normal 531a of facet 531.

When the surface normal 531a has been determined, the method comprises estimating the reverse surface normal 531b of the surface normal 531a as illustrated in Figure 6. By using the reverse surface normal 531b, the method comprises setting the optimal view direction to corresponds to the reverse surface normal 531b and updating the model view in the graphical user interface to correspond to the set optimal view direction.

If the selected at least one facet 531 is not associated with the affected area, then the processing of determining the optimal view direction will be stopped, and the processor in such case is configured to de-select the chosen facet.

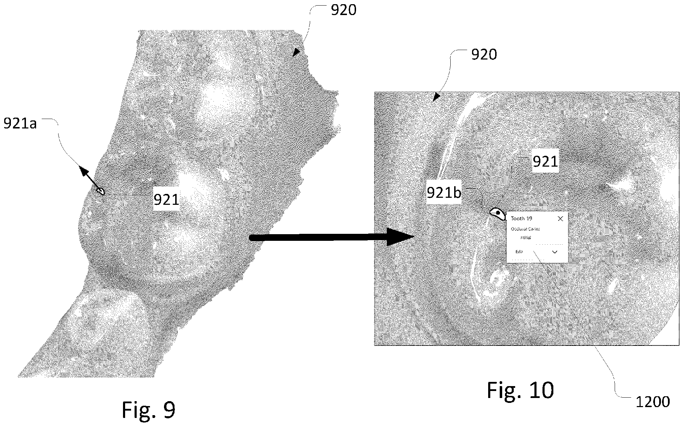

The output of the process according to the method described herein is illustrated in an example in Figure 9 and Figure 10, where the 3D model 920 in Figure 9 comprises affected area 921, with the estimated surface normal 921a. The reverse surface normal which is determined by the method described here is used to set the optional view direction and the subsequent update of the model view is illustrated in Figure 10. In Figure 10 it is clearly seen that the 3D model 920 is seen from a direction, where the affected area 921 can be seen in full, which ensures that the dentist may convey accurate information to the patient. In Figure 10, the estimated reverse normal vector 921b is illustrated as a dot to exemplify that the view direction has been updated.

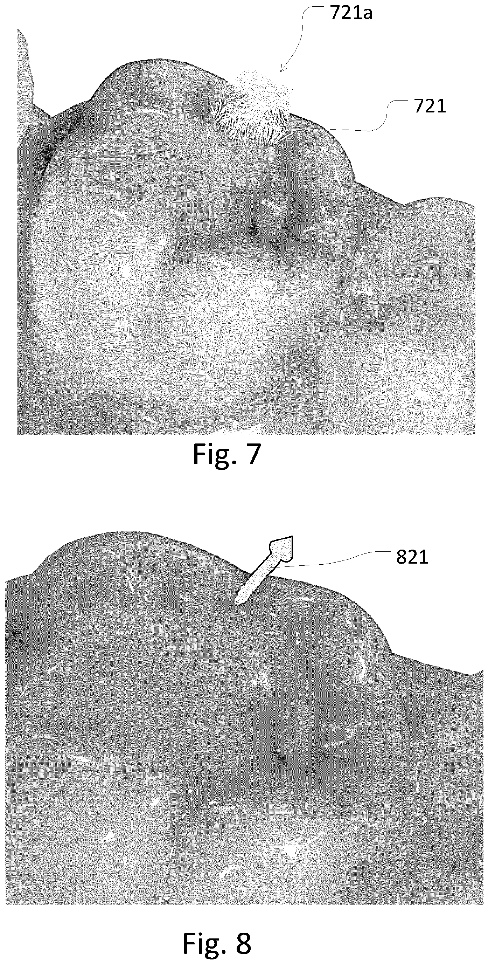

Referring now to Figure 7, an example is shown where the surrounding facets around an affected area are taken into consideration when determining the optimal lesion view direction. That is the method is configured to determining surface normals 721a for the one or more facets forming part of the affected area 721. The method provides for calculating an average of the surface normal and estimating the reverse normal of the averaged surface normals. This further allows the method to provide for setting the optimal lesion view direction as the reverse normal and updating the 3D model view in the graphical user interface to correspond to the set lesion view direction. An example of the determined average surface normal 821 can be seen in Figure 8. By using the surrounding facets, the optimal view direction estimation is improved, as it is based on an average normal 821 for the view direction as illustrated in Figure 8 rather than a single facet or point. The better optimal view direction is obtained from the average calculations as this provides for using the mesh curvatures of the affected area to move the view direction in a optimal direction for viewing the affected area.

In an example, the reverse normal vector is determined by normalizing the averaged surface normals form the lesion affected area facets and multiplying with -1.

In an example, the surrounding facets are chosen with flood fill using a distance algorithm, that is measuring the distance between facet mid points and if the distance is within the distance threshold, it takes that facet normal into consideration as well. Accordingly, the method comprises determining facets surrounding an affected area to determining the average of the surface normals within the affected area and the surface normals of facets surrounding the affected area. The surrounding facets are determined by applying a flood filling approach to calculate a distance between facet mid points of facets surrounding an affected area to facets within the affected area and applying a set threshold to determining if a surrounding facet should be included in the averaging or not.

In an example the method furthermore comprises checking if the affected area or parts thereof are occluded by other parts of the 3D model. Such an occlusion would not provide an accurate view of the model why it is considered to provide for a solution that takes into account any occluded area of a lesion affected area when setting the optimal view direction. Accordingly, in an example the method therefore provides for determining a lesion occlusal view configured as a non-lesion area of tooth and/or gingiva causing occlusion of at least a part of a lesion affected area, wherein the lesion occlusal view is determined by determining one or more boundary control points of a lesion affected area; estimating a ray from at least one spline control point and determining if the set optimal lesion view direction is occluded by the non-lesion area of tooth; and returning the set optimal lesion view direction if no occlusion is determined. The returned optimal lesion view direction is then used for setting the camera view from which the lesion affected area can been seen.

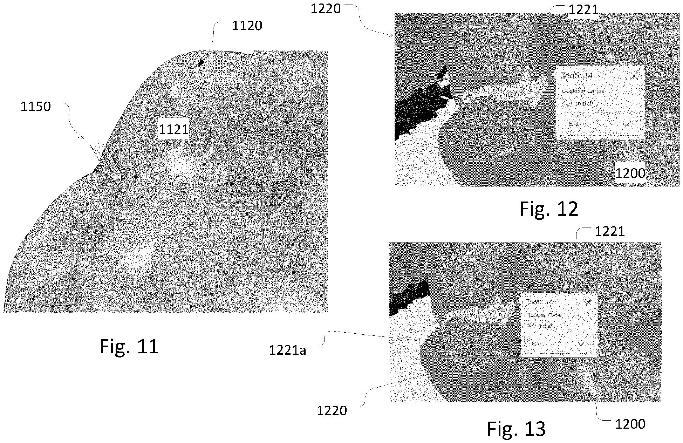

An example according to finding occlusion views can be seen in Figure 11. Here a 3D model 1120 comprising an affected area 1121 is illustrated. The affected area comprises a boundary representation, in the example illustrated as a spline encircling the boundary, wherein the boundary representation comprises control points. For each of the control points, a ray 1150 (illustrated as a plurality of rays 1150) may be estimated. In case any of the illustrated rays 1150 hits an occluding part of the 3D model 1120 when viewed from the view direction, the processor is configured to determine that the control point is occluded. This ensures that at least the boundary of an affected area is evaluated to determine if all parts of the boundary of the affected area can be seen from a current camera view.

In a further processing step, the method may also comprise evaluating not only the boundary areas as just described, but may be configured to evaluate any facet or point forming part of the affected area for occlusion. With this, the method may comprises determining for each facet, point or surface normal a ray and determine if the set view direction allows viewing the direction of the ray estimation for single points, facet or surface normals.

In an example, if a lesion occlusal view is determined, the method is configured to apply a transparency setting to the non-affected area of tooth and/or gingiva, wherein the transparency setting adjusts the opacity of the 3D model at the non-affected area of tooth and/or gingiva. This allows the 3D model to become more transparent in parts of the 3D model that are occluding the view of the lesion affected area.

In another example if a an occlusal view is determined, the method is configured to determining the optimal view direction by determining all facets within an affected area; determining the surface normal for each facet within the affected area; providing a weight to each of the surface normals, wherein the weights are determined based on the degree of occlusion which the surface normal is under; and calculating a weighted average of the surface normals to determine the reverse surface normal as the view direction based on the weighted average surface normals. This may be considered as an iterative approach, where the method is configured to determine for each surface normal associated with a facet if the facet is prone to occlusion and the degree of occlusion. When degree of occlusion for all facets in an affected area has been determined, the weighted average surface normal may be determined.

In a further example, the weighted average may be optimized to avoid that a fully occluded view is weighted high and potentially causes occlusion to another facet of the affected area. Such optimized weighted average calculation may according to the method described herein be determined by an iterative approach, where the weights are changed during the processing to allow for all facets being seen from the most optimal view thereby avoiding full occlusion of any of the facets in an affected area.

In a further example, the method comprises determining the weights not only by the degree of occlusion, but also by level of importance to the affected area. That is, if a lesion affected area comprises two levels of severity of a dental condition, it may be important that the more severe part of the affected area is clearly seen rather than the full affected area. Therefore, the method may be configured to determining if an affected area comprises more than one severity of a dental condition, and subsequently encoding a facet with the more severe facets with a higher weight to ensure that the facets associated with the higher weights counts higher when calculating the average surface normals. This way it is ensured that the facets associated with a more important area, such as a high severity dental condition becomes the focus of the view direction.

Figures 12 and 13 illustrate an example situation where an affected area comprises an occluded area, wherein the occluded area is part of the tooth. In Figure 12, an affected area 1221 of a 3D model 1220 is seen. The affected area 1221 when seen from the view in Figure 12 does not reveal that the affected area 1221 extends to areas of the tooth which cannot be seen in Figure 12. With the transparency setting example just described, the method comprises adjusting the 3D model 1220 to allow determination of an affected area and a subsequent change to areas occluding the affected area, wherein the changing of the 3D model comprises adjusting a transparency of the parts of the 3D model occluding the lesion affected area. This can be seen in an example in Figure 13, where 3D model 1220 of Figure 12, comprising affected area 1221 comprises an area 1221a which are occluded by a part of the tooth when seen in Figure 12, but which with the method described herein can be made transparent to allow the affected area to appear as seen in Figure 13.

Using a transparency setting as well as the iterative approaches explained to find the optimal view direction may as is apparent be combined to allow optimal affected area views.

To ensure that the affected area remains in focus of the camera according to the set optimal view direction when the dentist rotates, zoom or pan the model view, the method provides for setting the center of rotation while keeping the set optimal view direction. Accordingly, the method comprises setting a center of rotation of the affected area by determining a bounding box of at least one tooth enclosing all facets of the affected area; and calculating the center of the tooth and setting the center of the tooth as the center of the affected area.

In an alternative the center of rotation may be determined by determining the mass of the affected area and setting the center of rotation as the mass of affected area. In an embodiment, the center of mass of an affected area may be construed as the average position of all the vertex positions that form the facets belonging to the affected area. As an alternative, the center of mass may be calculated as an average of facet midpoints. In a further alternative, the center of mass may be calculated using an average of the control points of the boundaries. The center of mass calculation may be influenced by the density of points in a particular location. For example, a cube with 8 points is used, both the geometrical center and the center of mass will be at the center of the inside of the cube. But if one side of the cube had a large number of points on it, and everything else stayed the same, the center of mass will be closer to the beforementioned side, while the center of geometry will stay the same.

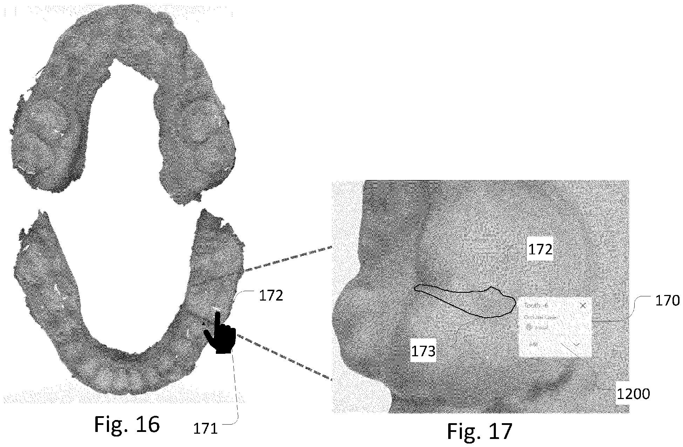

To allow inspection of an affected area as chosen by the user input encoding for the position representation, the method comprises generating an affected area control and rendering the affected area control in the graphical user interface together with at least a part of the 3D model comprising the affected area. An example of an affected area control can be seen in Figure 16 and 17, which illustrates the processing steps applied by the method when a user input is received by the processor. Accordingly, the method comprises generating an affected area control 170 upon receiving a user input 171 to a facet of the affected area 172 and updating the graphical user interface with the rendering of an affected area control 170, as seen in Figure 16 and Figure 17. The affected area control 170 is configured to displaying at least one of a tooth number, diagnostic indication and/or a severity of for example a diagnostic indication associated with the affected area 172 chosen from the user input 171.

As seen in Figure 17, the affected area control may comprise rendering of a 2D box formed structure configured to receive from a processor metadata information, such as the mentioned tooth number, diagnostic indication and /or severity. The metadata may be stored in the processor as a result of background processing method steps, such as the detection of indications, tooth numbers, tooth type, severity of indication etc. as performed by for example trained neural networks and/or other algorithmic procedures.

Furthermore, the affected area control may in an embodiment be configured to display information regarding the chosen affected area, such as the presence of a filling, the tooth being identified as a crown restoration, a veneer, inlay-onlay presence and/or the presence of attachments indication previous treatment procedures applied to the selected surface area of e.g. tooth and/or gingiva.

Furthermore, as illustrated in Figure 16, the method comprises overlaying an affected area with an area indication identifying a specific region of e.g. a tooth which is affected by for example a detected condition and/or where a previous treatment process have been applied. The area indication comprises a coloring of the affected area, wherein the coloring of the affected area represents for example a severity in case of a dental condition, a filling, a crown region etc. depending on what have been identified in connection with a tooth and/or gingiva region. Upon receiving the user input 172, the method comprises automatically zooming into the model and applying the previously explained optimal view direction setting to align the surface of the affected area with the focus view of the screen at which the model is represented, thereby ensuring an optimal view of the affected area. At the same time, the method comprises changing a transparency setting of overlayed color of the surface of the affected area, thereby rendering a 3D model with the affected surface area being encircled by the area indication, while leaving the inside of the area indication in the shape, form and content of the originally obtained 3D model. This can be seen in the change from Figure 16 to Figure 17, where it is schematically illustrated that when user input 171 encodes for surface affected area172, comprising a coloring of the surface affected area, the model is updated to the view illustrated in Figure 17. Here it is clearly seen that a rotation, translation and/or zoom has been applied to the 3D model, where surface affected area 172 can be viewed more from an optimal view, and where the entire surface affected area can be seen. To allow the dentist to assess the real data obtained from the intra-oral scan data, the method as explained as the same time ensures to generating a non-overlayed affected area, leaving only the affected surface area encircled by the area indication coloring 173. As also explained, at the same time the method provides for generating the affected area control 170 comprising information on the selected affected area obtained by stored meta data. This all together allows a dentist to easily inspect the affected area and convey any relevant information concerning the affected area for example to a patient. When the dentist then selects any region outside of the affected area, the method comprises updating the 3D model view to a view where the entire model can again be seen.

When the dentist is for example in the view illustrated in Figure 17 and explained above, it is important that the dentist can interact with the model by for example zooming, panning, rotating etc. the model to allow different views of the affected area. To support such zooming, panning and rotating and ensuring that the affected area control 170 does not obstruct any view of the affected area 172, when the model is for example rotated, the method comprises generating the affected area control 170 in close proximity and/or connected to the affected area. Furthermore, preferably the method comprises to rendering to the affected area control in a non-occluding manner in relation to the affected area.

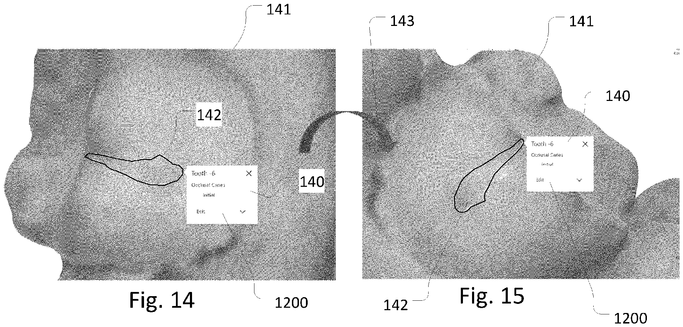

To ensure a non-obstructing view of the affected area by the area affected control, the method comprises determining and utilizing one or more control point points of boundary encircling the affected area to positioning the area affected control at a non-obstructing position. This process is best illustrated in Figure 14 and Figure 15, wherein Figure 14 illustrates 3D model 141 in a schematically example zoomed in as previously explained in relation to Figure 16 and 17 after an affected area 142 have been chosen by a dentist. As can be seen in Figure 14, the affected area control 140 is rendered at the right most position of the affected area 142 allowing an unobstructed view of the affected area 142. Upon receiving an input corresponding e.g. to a rotation of the 3D model 141, as illustrated by arrow 143, the 3D model 141 is updated accordingly to follow the path of rotation input by the user, as seen in Figure 15. This process activates the method described herein to updating at the same time the rendered positioning of the affected area control 140 into a new right most position in of the control points of a boundary encircling the affected area 142. This way, it is ensured that the area affected control does not block the view of the affected area allowing easy inspection of the affected area while moving the 3D model around.

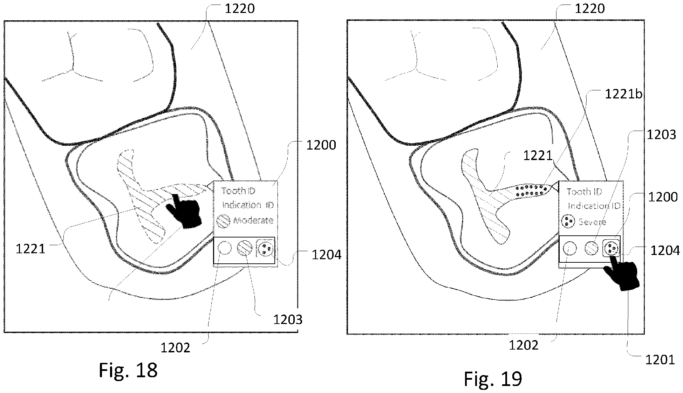

As seen in the Figures described herein, the affected area control comprises interactive elements, such as for example an "Edit" interactive element 1200 as seen in Figures 10, 12, 13, 14, 15 and 17. The interactive element 1200 is configured to be controlled by a user through the graphical user interface. That is, the method comprises receiving at least one user input to an interactive element 1200, wherein the user input encodes a change parameter to the diagnostic indication and/or severity; and retrieving the change parameter by the processor causing the method to perform the step of updating the diagnostic indication and/or severity in accordance with the change parameter. The updated change of indication and/or severity may according to the method be used to displaying an updated representation of the diagnostic indication and/or severity on the 3D model in the graphical user interface. This is illustrated schematically in Figure 18 and Figure 19, wherein a user selection 1201 of the "Editing" interactive element 1200 causes the method to generate a change parameter setting comprising one or more settings 1202, 1203, 1204, wherein at least one of the one or more settings 1202, 1203, 1204 corresponds to for example a severity of the affected area. In the case illustrated in Figure 18, the affected area is for example moderate severity, which is indicated by the dotted line in affected area control comprising the editing interaction element 1200, as well as in the 3D model 1220 where the affected area is also marked with a dotted line. Upon receiving a user input to the editing interactive element 1200, the method comprises generating one or more other setting parameters, as illustrated in Figure 19. Here a user input 1201 may encode for a changed setting parameter, as illustrated, encoding for a change to the affected area of the 3D model 1220. Accordingly, the method comprises receiving the user input 1201 encoding for a change for example a diagnostic indication of the lesion affected area and/or a change in severity 1204, wherein the change parameter encodes for a color change 1221b of the surface affected area 1221 in the 3D model 1220. This change may be relevant for the entire surface affected area 1221 or encode for only a part 1221b of the surface affected area.

With all of the different examples and embodiments described herein a solution has been provided for, which ensures fast assessment and conveying of dental health assessment within digital dentistry that allows optimized treatment processes of patients.

According to examples described herein, computer program executed by e.g. a processor shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

Although some embodiments and described examples have been described and shown in detail, the disclosure is not restricted to such details, but may also be embodied in other ways within the scope of the subject matter defined in the following claims. In particular, it is to be understood that other embodiments and examples described may be utilized, and structural and functional modifications may be made without departing from the scope of the present invention.

Benefits, other advantages, and solutions to problems have been described herein with regard to specific embodiments and examples. However, the benefits, advantages, solutions to problems, and any component(s)/ unit(s) that may cause any benefit, advantage, or solution to occur or become more pronounced are not to be construed as critical, required, or essential features or components/ elements of any or all the claims or the invention. The scope of the invention is accordingly to be limited by nothing other than the appended claims, in which reference to a component/ unit/ element in the singular is not intended to mean "one and only one" unless explicitly so stated, but rather "one or more." A claim may refer to any of the preceding claims, and "any" is understood to mean "any one or more" of the preceding claims.

It is intended that the structural features of the devices described above, either in the detailed description and/or in the claims, may be combined with steps of the method, when appropriately substituted by a corresponding process.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element but an intervening elements may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method is not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

The claims are not intended to be limited to the aspects shown herein, but is to be accorded the full scope consistent with the language of the claims, wherein reference to an element in the singular is not intended to mean "one and only one" unless specifically so stated, but rather "one or more." Unless specifically stated otherwise, the term "some" refers to one or more.

## Claims

1. Computer-implemented method for generating an optimal view direction of an affected area in a 3D model of intra-oral scan data, the method comprising:
- rendering a 3D model in a graphical user interface of a computer display, wherein the 3D model comprises one or more affected areas;
- receiving by a processor a user input to the graphical user interface, wherein the user input encodes a position representation in the rendered 3D model;
- retrieving the position representation and determining if the position representation is associated with an affected area, wherein if the position representation is associated with an affected area the method comprises:
- determining an optimal view direction of the affected area using the position representation by:
- determining a surface normal of the position representation;
- estimating the reverse normal of the surface normal; and
- setting the optimal view direction as the reverse normal and updating the 3D model view in the graphical user interface to correspond to the set optimal view direction.

2. The method according to claim 1, wherein the position representation encodes for a selection of at least one facet.

3. The method according to claim 2 further comprising evaluating if the selected at least one facet forms part of the affected area of a tooth and/or a gingiva area of the 3D model.

4. The method according to claim 2, wherein if the facet does not form part of an affected area the processor is configured to de-select the facet.

5. The method according to claim 2, wherein the surface normal is calculated from at least one facet and wherein the reverse normal is estimated from the calculated surface normal.

6. The method according to claim 1, wherein the affected area comprises one or more facets, wherein setting the optimal view direction comprises
- determining surface normals for the one or more facets forming part of the affected area;
- calculating an average of the surface normals;
- estimating the reverse normal of the averaged surface normals; and
- setting the optimal view direction as the reverse normal and updating the 3D model view in the graphical user interface to correspond to the set optimal view direction.

7. The method according to claim 6, wherein the reverse normal vector is determined by normalizing the averaged surface normals from the affected area facets and multiplying with -1.

8. The method according to any of the previous claims, wherein setting the optimal view direction further comprises:
- determining facets surrounding the affected area to determining the average of the surface normals within the affected area and the surface normals of facets surrounding the affected area, wherein the surrounding facets are determined by:
- applying a flood filling approach to calculate a distance between facet mid points of facets surrounding the affected area to facets within the affected area and applying a set threshold to determining if a surrounding facet should be included in the averaging or not.

9. The method according to any of the previous claims, wherein the affected area comprises a boundary encircling the affected area, wherein the boundary comprises control points.

10. The method according to any of the previous claims, wherein the method comprises determining an area affected occlusal view configured as a non-affected area of tooth and/or gingiva causing occlusion of at least a part of the affected area, wherein the area-affected occlusal view is determined by
- determining one or more boundary points of the affected area;
- estimating a ray from at least one boundary point and determining if the set optimal view direction is occluded by the non-affected area of tooth and/or gingiva; and
- returning the set optimal view direction if no occlusion is determined.

11. The method according to claim 10, wherein if the area affected occlusal view is determined applying a transparency setting to the non-affected area of tooth and/or gingiva, wherein the transparency setting adjusts the opacity of the 3D model at the non-affected area of tooth and/or gingiva.

12. The method according to any of the previous claims, further comprising setting a center of rotation of the affected area by determining a bounding box of at least one tooth enclosing all facets of the affected area;
calculating the center of the tooth and setting the center of the tooth as the center of the affected area.

13. The method according to any of the previous claims, further comprising generating an area affected control and representing the area affected control in the graphical user interface, wherein the area affected control is generated upon receiving a user input to a facet of the affected area, and wherein the area affected control is configured to display tooth number, diagnostic indication and/or a severity of a diagnostic indication associated with the facet of the affected area.

14. The method according to any of the previous claims, wherein the method is configured to receive a user input of a non-affected area causing the processor to re-orient the view direction to the center of location of a global coordinate system of the 3D model.

15. A system for generating 3D models and inspecting diagnostic data, wherein the system comprises
- an intra-oral scanner configured to obtain scan data;
- a computing device operatively communicating with the scanner, the computing device configured to:
- generate a 3D model of the intra-oral scan data;
- identifying one or more affected areas in the intra-oral scan data;
- rendering a 3D model from the generated 3D model of intra oral scan data in a graphical user interface of a computer display, wherein the 3D model comprises one or more affected areas;
- receiving by a processor a user input to the graphical user interface, wherein the user input encodes a position representation in the rendered 3D model;
- retrieving the position representation and determining if the position representation is associated with an affected area, wherein if the position representation is associated with an affected area, the computing device is configured to
- setting an optimal view direction from where the affected area is displayed in the graphical user interface using the position representation, wherein setting the optimal view direction comprises:
- determining a surface normal of the position representation;
- estimating the reverse normal of the surface normal; and
- setting the optimal view direction as the reverse normal and updating the 3D model view in the graphical user interface to correspond to the set view direction.
